# EUROPEAN PATENT SPECIFICATION

(11) **EP 1 879 552 B1**
(45) Date of publication and mention of the grant of the patent: **17.02.2016**
(21) Application number: 06748398.2
(22) Date of filing: 14.03.2006
(51) Int. Cl.: A61K 9/70, A61L 27/34, A61L 27/54, A61K 9/20, A61K 47/48, A61L 31/16, A61L 31/10, A61L 29/16, A61L 29/08

(54) **DRUG DELIVERY COMPOSITIONS COMPRISING A POLYELECTROLYTE COMPLEX WITHIN A POLYMERIC MATRIX**
ARZNEIMITTELABGABEZUSAMMENSETZUNGEN UND DAZUGEHÖRIGE VERFAHREN
COMPOSITIONS A LIBERATION D'UN PRINCIPE ACTIF ET LES PROCEDES CORRESPONDANTS

(30) Priority: 14.03.2005 US 661799 P
(43) Date of publication of application: 23.01.2008
(73) Proprietor: Biotegra, Inc., Lake Forest, CA 92630 (US)
(72) Inventor: HSU, Li-Chien, Mission Viejo, California 92691 (US); TONG, Sun-De, Irvine, California 92620 (US)
(74) Representative: Müller-Boré & Partner Patentanwälte PartG mbB
(86) International application number: PCT/US2006/009431
(87) International publication number: WO 2006/099514

(56) References cited:
- WO-A-03/063799
- US-A- 5 863 554
- US-A1- 2003 235 589
- US-B1- 6 193 970

## Description

### BACKGROUND

The ability to controllably deliver therapeutic or prophylactic agents to a specific target site within the body has been a goal in the field of drug delivery as a controlled level of drugs in the body provides the most effective prophylactic or therapeutic treatment for preventing or treating illnesses. Accordingly, various different types of drug delivery modes have been developed to deliver drugs to a particular site in or on the body.

For example, in a matrix drug delivery system, drug molecules are dissolved or dispersed in a polymer matrix. The drug molecules are released to the external environment by diffusing through the polymer matrix to the surface of the matrix. The release rate for a drug is generally dependent upon the polymer used in the drug delivery system. For non-biodegradable and biodegradable polymers, the release rate is generally proportional to the diffusion coefficient of the drug molecules dissolved in the polymer. For biodegradable polymers, the release rate of a drug is also dependent upon the degradation rate of the polymers. Additionally, various additives, such as salts, metal cations, and lipids, have been incorporated into a polymeric matrix in order to further regulate the drug release.

US 2003/235589 describes embodiments where drugs are encapsulated within a chitosan/gelatin nanosphere, to which is coupled an anti-human Type II collagen monoclonal antibody. In this case, chitosan is the polyelectrolyte, whereas gelatin functions as polymer matrix. No material of opposite charge to chitosan is involved in the "polyelectrolyte" or chitosan, however.

WO 03/063799 discloses a chondroprotective composition suitable for systemic delivery, such as by intravenous, intramuscular, subcutaneous or inhalation administration. The IGF receptor agonist, IGF-1 is separately encapsulated within a chondroiten-6-sulfate/gelatin nanosphere to which is also coupled an anti-human aggrecan monoclonal antibody. Thus, WO 03/063799 provides information on a composition for controlled IGF-1 release, and therewith on the method for regulating the release of said IGF-1. In this example of WO 03/063799, chondroiten-6-sulfate is the polyelectrolyte; and gelatin is considered as the polymer. No material of opposite charge to chondroiten-6-sulfate is involved, however.

US 6,193,970 discloses embodiments where high doses of GM-CSF are encapsulated in cell-sized gelatin-chondroiten-6-sulfate microspheres, in order to be mixed with irradiated tumor cells prior to subcutaneous injection. However, no material of opposite charge to chondroiten-6-sulfate is involved.

In US 5,863,554 insulin is combined with chitosan and starch in microspheres. However, in said document chitosan is the polyelectrolyte and starch serves as the polymer. No polyelectrolyte complex is disclosed, neither in the description nor in the example.

While the drug delivery system may properly release drugs, complications relating to stability and compatibility may develop when the drug delivery system remains in contact with tissue or biological fluids for extended periods of time. For example, a drug delivery system including synthetic polymers may induce severe coagulation activation, adverse cellular, humoral, and inflammatory responses when contacted with blood or tissue. These undesirable responses resulting from blood/tissue and material interactions may significantly affect the safety and efficacy of an implanted medical device and may defeat the purpose of the implanted device.

Thus, there is a continuing need for a polymer matrix that is capable of releasing a wide range of therapeutic amounts of drug and possesses desirable blood and/or tissue compatibility.

### BRIEF SUMMARY

According to the present invention, there is provided a drug delivery composition comprising:
a polymer;
one or more drugs dispersed within the polymer; and
a bioactive polyelectrolyte complex dispersed within the polymer that regulates the release of the drugs from the polymer, wherein the bioactive polyelectrolyte complex comprises a polyelectrolyte and an oppositely charged component, wherein the polyelectrolyte and/or the oppositely charged component is bioactive, or wherein the bioactive polyelectrolyte complex is formed from positively-charged and negatively-charged polyelectrolytes.

Briefly, and in general terms, various embodiments are directed to a drug delivery composition that regulates the release of a drug. In one embodiment, the drug delivery composition includes a polymer, and one or more drugs and a bioactive polyelectrolyte complex dispersed within the polymer where the polyelectrolyte complex regulates the release of the drugs from the polymer. The bioactive polyelectrolyte complex comprises a polyelectrolyte and an oppositely charged component, wherein the polyelectrolyte and/or the oppositely charged component is bioactive.

In addition to a drug delivery composition, various embodiments of a medical device are disclosed herein. According to one embodiment, the medical device includes one or more surfaces, wherein at least one surface includes the above biocompatible drug delivery composition. The drug delivery composition includes a polymer, and one or more drugs and a bioactive polyelectrolyte complex dispersed within the polymer where the polyelectrolyte complex regulates the release of the drugs from the polymer. The bioactive polyelectrolyte complex comprises a polyelectrolyte and an oppositely charged component, wherein the polyelectrolyte and/or the oppositely charged component is bioactive.

Additionally, various methods of regulating the release of one or more drugs are disclosed herein. According to one method, a drug delivery system is provided and includes one or more drugs and a bioactive polyelectrolyte complex dispersed within the polymer matrix. The polyelectrolyte complex comprises a charged, biologically active component and an oppositely charged component. The release rate of the drugs from the drug delivery system is regulated by adjusting the concentration of the bioactive polyelectrolyte complex. Additionally, the bioactive electrolyte complex elutes from the drug delivery system to provide an additional therapeutic or diagnostic effect.

Other features and advantages will become apparent from the following detailed description, taken in conjunction with the accompanying drawings, which illustrate by way of example, the features of the various embodiments.

### BRIEF DESCRIPTION OF THE DRAWINGS

FIG. 1 graphically depicts the release rate of a water-insoluble drug from one embodiment of a drug delivery composition having a bioactive polyelectrolyte complex versus the release rate of a water-insoluble drug from a control sample not having a bioactive polyelectrolyte complex;
FIG. 2 graphically depicts the release rate of a drug from various embodiments of a drug delivery composition having different bioactive polyelectrolyte complex concentrations;
FIG. 3 graphically depicts the release rate of a water-soluble drug from one embodiment of a drug delivery composition having a bioactive polyelectrolyte complex versus the release rate of a water-soluble drug from a control sample not having a bioactive polyelectrolyte complex;
FIG. 4 graphically depicts the release rate of a bioactive polyelectrolyte complex from one embodiment of a drug delivery composition; and
FIG. 5 graphically depicts the release rate of a water-insoluble drug from two embodiments of a drug delivery composition having different types of polymers.

### DETAILED DESCRIPTION

Various embodiments disclosed herein are directed to drug delivery compositions for modulating the release of biologically active agents (also referred to herein as "drugs"). Generally, the composition comprises a biologically active agent and a polyelectrolyte complex that are dissolved or dispersed within a polymeric matrix. The polyelectrolyte complex allows the drug release rate to be controllably altered by adjusting the relative concentrations of the polyelectrolyte complex. The polyelectrolyte complex is capable of modulating the initial burst as well as the subsequent sustained release of the biologically active agents. Additionally, the polyelectrolyte complex is biologically active thereby providing desirable blood and/or tissue compatibility. Furthermore, the bioactive polyelectrolyte complex may be released from the polymeric matrix and thereby providing additional prophylactic or therapeutic effects independent of the biologically active agent. The polyelectrolyte complex also modifies the physical properties of the polymeric matrix. For example, the presence of the polyelectrolyte complex contributes to making the polymers more flexible and, thus, suitable for applications involving flexion and expansion.

Generally, the drug delivery composition for a biologically active agent includes at least one polymer, at least one bioactive polyelectrolyte complex (BPEC), and at least one therapeutic agent, wherein the therapeutic agent(s) are dissolved or dispersed in a mixture of the polymer and polyelectrolyte complex. A polyelectrolyte is defined as a compound bearing two or more charged groups. The bioactive polyelectrolyte complex comprises a polyelectrolyte and an oppositely charged molecule. In these various embodiments, complexing a polyelectrolyte with an oppositely charged molecule forms a complex that may be combined with polymers.

Non-limiting examples of polyelectrolytes used with the drug delivery system include, but are not limited to, sodium salt of double and single stranded DNA; glycosaminoglycans such as, but not limited to, dextran sulfate, chondroitin sulfate, dermatan sulfate, dermatan disulfate, heparan sulfate, keratan sulfate, hyaluronic acid, heparinoid, heparin; anionic polysaccharides; xanthan gum; carrageenan; polyacrylic acid; poly(styrene sulfonate); polyamino acids such as poly(L-lysine); poly (L-arginine); poly (glutamic acid); poly(L-histidine); oligopeptides; insulin; monoclonal antibodies; protamine sulphate; polyhexamethylene biguanide; chitosan; polyethyleneimine; polydimethyldiallyammonium chloride; poly(N-methyl-4-vinyl-pyridinium); poly (ionene-6,3); quaternized polymers or copolymer of dimethylaminoethyl methacrylate; and other polymers or copolymers containing charged groups.

Non-limiting examples of small molecules bearing opposite charge that is combined with the bioactive polyelectrolyte complex include, but are not limited to, retinoic acid, compounds containing amino or amine functional groups (e.g., primary, secondary, tertiary, and quaternary amines), such as amitriptyline, penicillian, amoxicillian, tyramine, hexadimethrine bromide, tridodecyl amine, trimethyltetradecylammonium chloride, alkyldimethylbenzyl ammonium chloride, tridodecylmethyl ammonium chloride, or other cationic, anionic, and amphoteric surfactants.

As previously mentioned, the polyelectrolyte complexes may also possess at least one component that is biologically active independent of the drugs provided in the drug delivery system. Accordingly, the polyelectrolyte and/or the complexing agent may be bioactive. For example, heparin, heparinoids, and heparan sulfates are established anti-coagulants. Alternatively, dermatan sulfate may be used to accelerate the interaction between heparin co-factors and thrombin. In another embodiment, retinoic acid may be used to proliferate and differentiate epithelial tissue as well as to inhibit malignant tumors.

The following are specific yet non-limiting examples of polyelectrolyte complexes that may be used in a drug delivery composition: a complex of Vitamin A acid (retinoic acid) and poly(L-lysine); insulin and poly(methacrylic acid); insulin and hyaluronic acid; and heparin and dimethylalkylbenzyl ammonium chloride.

As previously mentioned, a controlled release rate of the biologically active agent from the composition may be adjusted by varying the relative concentration of the polyelectrolyte complex. A controlled release of a biologically active agent, as defined herein, is a release of a biologically active agent from the polymeric matrix including a bioactive polyelectrolyte complex. The presence of the polyelectrolyte complex in the polymeric matrix alters the one or more of the drug release characteristics such as initial drug release level, subsequent drug release levels, the total amount of drug released, and/or the extent of the release period as compared to a polymeric matrix not containing a polyelectrolyte complex.

Depending on the physical properties (e.g., solubility) of the biologically active agent, the bioactive polyelectrolyte complex may have different effects on the drug release rate. For example, the release rate of a water insoluble drug may be increased in the presence of a bioactive polyelectrolyte complex. In contrast, the release rate of a water-soluble drug may be reduced in the presence of a bioactive polyelectrolyte complex. As those skilled in the art will appreciate, a suitable concentration of the bioactive polyelectrolyte complex that will modulate release of the biologically active agent from the polymeric matrix and improve biocompatibility depends upon the physical and chemical properties of the polymer, the polyelectrolyte complex, and the biologically active agent. In one embodiment, suitable amounts of bioactive polyelectrolyte complex range from approximately 0.1% (w/w) to approximately 90% (w/w). However, those skilled in the art will appreciate that any range or concentration of the bioactive polyelectrolyte complex may be used in the drug delivery system.

In addition to providing improved biocompatibility and modulating the drug release, the bioactive polyelectrolyte complexes may also modify the physical properties of the polymer matrix. For example, incorporating the bioactive polyelectrolyte complex into the polymer matrix lowers the glass transition temperature of the polymers matrix. (See Example 6 below.) As those skilled in the art will appreciate, changes in the physical properties of the polymeric matrix may be useful in those applications where an implant moves, flexes, vibrates, or stretches during and/or after implantation. Additionally, the bioactive polyelectrolyte components may also modify the hydrophilicity and/or hydrophobicity of the polymer matrix such that the polymer is more or less compatible (e.g., swellable, permeable, or wettable) in an aqueous or organic solvent. Furthermore, the bioactive polyelectrolyte complex may alter the diffusion rate of the polymer matrix. That is, the bioactive polyelectrolyte complex may increase or decrease the diffusion rate of a drug from the polymer matrix.

Another component of the drug delivery composition is the polymers that comprise the polymeric matrix. Generally, any polymer or mixture (e.g., blend or copolymer) of polymers, whether biodegradable or non-biodegradable, that is physically combinable (e.g., mixable, dissolved, or dispersed) with the polyelectrolyte complexes and the drugs are suitable for the drug delivery compositions disclosed herein. Suitable biodegradable polymers include, but are not limited to, poly(lactic acid)s, poly(glycolic acid)s, poly(lactic acid-co-glycolic acid)s, polyanhydrides, polyorthoesters, polyetheresters, polycaprolactone, polyesteramides, blends and copolymers thereof. Non-biodegradable polymers include, but are not limited to, polyolefins, polystyrene, polyvinyl acetate, acrylic polymers, polyethers, fluoropolymers, polyesters, polyamides, polyurethanes, silicone polymers, polydienes, cellulose and its derivatives, blends and copolymers thereof. Other examples include cross-linkable water-soluble polymers, such as polyethylene glycol, polyvinyl alcohol, polyvinyl pyrrolidone, gelatin, and starch.

The following are specific yet non-limiting examples of polymers that may be used in a drug delivery composition. For example, poly(alkyl methacrylate) based homopolymers or copolymers may be used to form the polymeric matrix. Alternatively, other polymers that may be used to form the polymeric matrix include poly(lactic acid)s, poly(lactic acid-co-glycolic acid)s, polyalkylmethacrylate, poly(ethylene- vinyl acetate), and copolymers of styrene and diene.

Another component of the drug delivery composition is the biologically active agent or drug that are controllably released from the composition. The biologically active agents, as defined herein, are those agents possessing therapeutic, prophylactic, or diagnostic properties in vivo. Examples of suitable therapeutic and/or prophylactic biologically active agents include anti-inflammatory agents, anti-proliferative agents, antineoplastic agents, anti-mitotic agents, anti-migratory agents, agents affecting extracellular matrix production and organization, agents promoting healing and re-endothelialization, anti-coagulants, anti-thrombotic agents, vascular cell growth promoters, vascular cell growth inhibitors, cholesterol-lowering agents, vasodilating agents, anesthetic agents, lubricants or humectants, anti-glaucoma agents, anti-cataract agents, anti-crustation agents, analgesics, antiasthmatics, antibiotics, antidepressants, antidiabetics; antifungal agents, antihypertensive agents, anticancer agents, antianxiety agents, immunosuppressive agents, antimigraine agents, antianginal agents; antipsychotic agents; antimanic agents; antiarthritic agents, anti-gout agents; anticoagulants, thrombolytic agents, fibrinolytic agents; antiplatelet agents; anticonvulsants; antiparkinson agents; antihistamines; antiviral agents, antimicrobials, sedatives, hemorheologic agents, bronchodilators, steroidal compounds, hypoglycemic agents, hypolipidemic agents, proteins, nucleic acids, and vitamins. Diagnostic agents include, but are not limited to, contrasting agents such as, but not limited to, radioactive isotopes and radiopaque agents.

The following are specific yet non-limiting examples of drugs that may be used in the drug delivery composition that include, but are not limited to, ibuprofen, salicylic acid, ticlopidine, clopidogrel, prasugrel, glycoprotein IIbIIIa inhibitor, statins, heparin, hirudin, urokinase, streptokinase, tissue plasminogen activator, nitric oxide, angiotensin converting enzyme inhibitor, dexamethsone, sirolimus, everolimus, zatarolimus, pimecrolimus, tacrolimus, biolimus, paclitaxel, estradiol, tranilast, tripidal, angiopeptin, brimonidine, timiolol, carbonic anhydrase inhibitors, their analogs and derivatives.

As those skilled in the art will appreciate, an effective amount of the biologically active agent is incorporated within a polymeric matrix of the drug delivery composition. An effective amount of a biologically active agent is defined as a therapeutically or prophylactically effective amount, which can be determined by a person of ordinary skill in the art, taking into consideration factors such as, but not limited to, a patient's weight, age, physical condition, therapeutic, prophylactic or diagnostic goal, type of agent used, type of polymer used, and desired release rate (e.g., initial burst and subsequent release rate). Suitable amounts of the biologically active agent will range from approximately 0.01 % (w/w) to approximately 70% (w/w). However, those skilled in the art will appreciate that any range or concentration of the biologically active agent not specifically recited may be used in the drug delivery system.

In addition to drug delivery compositions, various embodiments disclosed herein are directed to medical devices capable of controllable delivery of a biologically active agent. According to one embodiment, the drug delivery compositions are incorporated into a medical device. For example, the drug delivery composition may be a film on the device or a portion of the device. Alternatively, the composition may be a coating that is bonded to one or more surfaces of the device. Generally, at least a portion of the medical device that is insertable or implantable into the body of a patient includes the drug delivery composition. In another embodiment, the drug delivery composition, itself, may be used to form into a medical device or a portion of a medical device.

As those skilled in the art will appreciate, the drug delivery composition may be applied to or formed into medical devices such as, but are not limited to, contact lenses, intraocular lenses, orofacial implants, bone cements, stents, stent grafts, urinary stents, intravascular or intervascular balloons, shunts, anastomosis devices, heart valves, catheters, guide wires, balloons, filters, vascular grafts, vascular patches, or intraluminal paving systems.

Various methods of forming a drug delivery composition are disclosed herein. The drug delivery composition is generally formed through a solvent evaporation process. The polymeric solution containing the bioactive polyelectrolyte complex and at least one biologically active agent is cast in a mold or applied to one or more surfaces of a device. The solvent is then evaporated to leave behind the drug delivery composition comprising a polymeric matrix, a bioactive polyelectrolyte complex, and at least one biologically active agent. As those skilled in the art will appreciate, the desired amounts of the biologically active agents, bioactive polyelectrolyte complex, and the polymer may be varied depending upon the desired drug release levels and time span of drug release.

According to one method, the drug delivery composition may be formed into a medical device. In this method, a polymer is dissolved in a solvent system to form a polymer solution. The bioactive polyelectrolyte complex is dissolved or dispersed in the polymer solution. The biologically active agent is then dissolved or dispersed in the polymer solution. Optionally, the biologically active agent may be dissolved or dispersed in the polymer solution at the same time the bioactive polyelectrolyte complex is added to the polymer solution. The polymer solution containing the bioactive polyelectrolyte complex and the biologically active agent is cast in a mold. The polymer solution is then solidified to form a polymeric matrix containing a dispersion of the bioactive polyelectrolyte complex and the biologically active agent.

According to another method, a suitable polymer is dissolved in a solvent to form a polymer solution. The bioactive polyelectrolyte is dissolved or dispersed within the polymer solution. As those skilled in the art will appreciate, a suitable means for dissolving the polyelectrolyte complex within a polymer solution may be achieved by dissolving both polymer and polyelectrolyte complex in a mutual solvent. Alternatively, the polyelectrolyte complex is added into the polymer solution and then through agitation or mixing to disperse the polyelectrolyte complex within the polymer solution. In either the dissolution or dispersion method, the polyelectrolyte complex may be added in the form of a solid or liquid before, during, or after the preparation of polymer solution. Alternatively, the polyelectrolyte complex may be dissolved or dispersed in a second solvent and this solution is then added to the polymer solution. As those skilled in the art will appreciate, the second solvent is suitable if it is miscible with the polymer solution.

According to one method, at least one biologically active agent is also added to the polymer solution separately from the addition of the bioactive polyelectrolyte complex. In one method, the biologically active agent is added directly into the polymer solution containing the bioactive polyelectrolyte complex. In another method, the biologically active agent is dissolved or dispersed in a solvent, which is also miscible with the polymer solution, and then this solution is mixed into the polymer solution.

As those skilled in the art will appreciate, the drug contained within the drug delivery composition may be administered to a human or other animal, by implantation or insertion as a medical device. The medical device may be made from the drug delivery composition. Alternatively, the drug delivery composition may be used as a film or coating on a device for subcutaneous, intramuscular, intraperitoneal, intradermal, intravenous, intra-arterial, intrathecal or intranasal applications. For example, the drug delivery composition is impregrated into or coated onto an intravascular device such as a stent. Alternatively, the drug delivery composition may be administered to a patient topically. The drug delivery composition may be included with a cream, hydrogel, patch, or the like.

In use, an implant or medical device including a drug delivery composition disclosed herein is delivered to a target site by any processes known or developed in the art. For example, a stent having the drug delivery composition is delivered to the vasculature via a catheter. Once implanted, the drug delivery composition is exposed to both physical stresses (e.g., flowing blood) and chemical agents (e.g., various enzymes and proteins found in the blood). Depending upon the relative concentrations of each component of the drug delivery composition, the biologically active agents and the bioactive polyelectrolyte complex are released from the medical device according to a desired drug elution profile designed in accordance with the teachings of this specification.

The various embodiments disclosed herein are illustrated by the following examples. These examples are provided for exemplification and are not intended to be limiting.

### EXAMPLE 1

The bioactive polyelectrolyte complex (BPEC) used in this example was a complex formed between chondroitin sulfate and alkyldimethylbenzyl ammonium chloride. Paclitaxel, a water-insoluble drug, was used as a model compound for the biologically active agent. Appropriate amounts of non-biodegradable polybutylmethacrylate, bioactive polyelectrolyte complex, and paclitaxel were dissolved in a solvent system of toluene and alcohol resulting in a bioactive polyelectrolyte complex content of 15%. A control solution was prepared using polybutylmethacrylate and paclitaxel, but did not include the bioactive polyelectrolyte complex.

A stainless steel wire was spray-coated with each of the solutions described above. The amount of solution sprayed on each wire was controlled so that the each wire contained about the same amount of the drug delivery matrix. The coated wire was then dried until a constant weight was reached. A film was formed on the stainless steel wire after the completion of the drying process.

To determine the drug release rate, each coated stainless steel wire was incubated in saline solution containing 1% Tween-80 at 37° C. Triplicate samples were tested. At various time points, an aliquot of the incubating solution was removed for analysis of the amount of drug released. The amount of solution removed was replaced with fresh solution. The amount of paclitaxel released into the incubating solution at each time point was determined by high pressure liquid chromatography (Supelco supelcosil LCF column with a mixture of acetonitrile and water as the mobile phase).

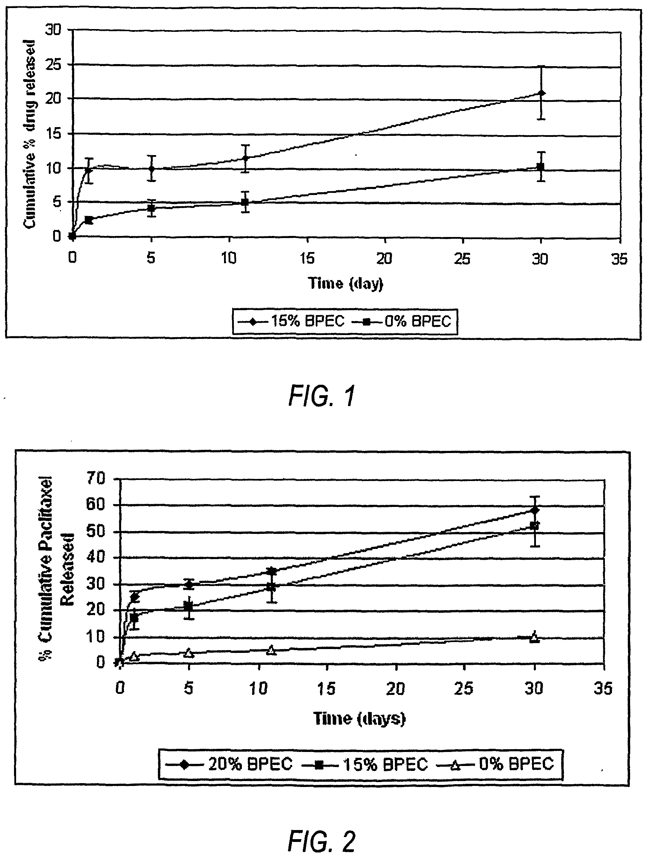

The cumulative amounts of paclitaxel released from the drug delivery systems having a bioactive polyelectrolyte complex content of 0% and 15% are shown in FIG. 1. As shown in FIG. 1, the presence of bioactive polyelectrolyte complex causes an increase in the initial drug release level, sustained drug release levels, and the total amount of drug released as compared to the control sample that did not include the bioactive polyelectrolyte complex.

### EXAMPLE 2

The sample preparation and analytical techniques were the same as those described in Example 1, except chondroitin sulfate was replaced with heparin. Appropriate amounts of non-biodegradable polybutylmethacrylate, bioactive polyelectrolyte complex, and paclitaxel were dissolved in a solvent system comprising toluene and alcohol in order to form drug delivery systems having a bioactive polyelectrolyte complex content of 15% and 20%, respectively, while the drug content remained constant at 7%. A control solution was prepared using polybutylmethacrylate and paclitaxel but did not include the bioactive polyelectrolyte complex.

A stainless steel wire was spray-coated with each of the solutions described above. The coated wire was then dried until a constant weight was reached. A film was formed on the stainless steel wire after the completion of the drying process. The amount of solution sprayed on each wire was controlled so that the each wire contained about the same amount of the drug delivery matrix as shown below:

| | 20% BPEC | 15% BPEC | 0% BPEC |
|---|---|---|---|
| weight gain of wire (n = 5) | 473 +/- 24 µg | 445 +/- 32 µg | 468 +/- 8.8 µg |

The drug release rates from compositions containing 0%, 15%, and 20% are shown in FIG. 2. As shown in FIG. 2, the presence of bioactive polyelectrolyte complex causes an increase in the initial drug release level, sustained drug release levels, and the total amount of drug released relative as compared to a control sample that did not include the bioactive polyelectrolyte complex. Additionally, as shown in FIG. 2, a higher content (20%) of the bioactive polyelectrolyte complex increased the drug release characteristics, including initial drug release level, sustained drug release levels, and the total amount of drug released as compared to a drug delivery system having 15% content of bioactive polyelectrolyte complex.

### EXAMPLE 3

The method of solution preparation was the same as that described in Example 2, except that Eosin Y was used as a model compound to represent the water-soluble drug. Using the same polymer and similar techniques as described in Example 2, aluminium disks were coated with a polymer matrix solution with and without the bioactive polyelectrolyte complex.

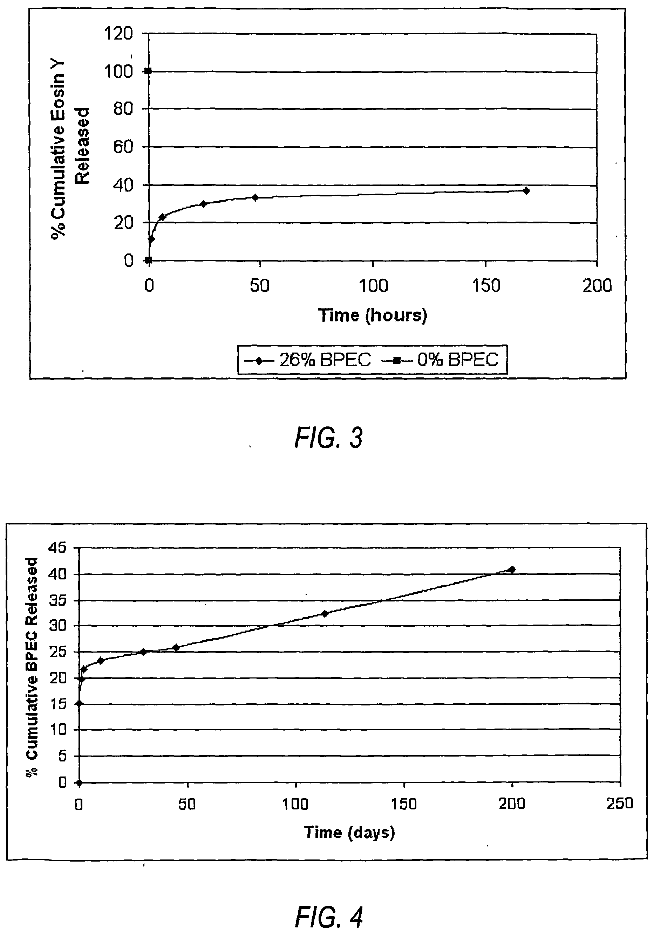

To determine the Eosin Y release rate, each coated disk was incubated with a known quantity of saline containing 1% Tween-80. The amount of Eosin Y released into the incubating solution was determined by UV/visible spectrophotometer at various time points. At each time point, an aliquot of the incubating solution medium was withdrawn and analyzed for Eosin Y content. The amount removed was replaced with fresh solution. The amounts of Eosin Y released from polymeric matrices with and without BPEC are shown in FIG. 3.

FIG. 3 illustrates the difference between a control sample and a sample having a BPEC. In the absence of bioactive polyelectrolyte complex, all of the Eosin Y is released immediately from the polymer matrix. That is, there was no modulation of drug release rate with a drug delivery system having only the polymer. In the drug delivery system having 26% bioactive polyelectrolyte complex, the initial Eosin Y release level, sustained Eosin release levels, and the total amount of Eosin released are substantially decreased and modulated by the presence of the bioactive polyelectrolyte complex. Furthermore, the bioactive polyelectrolyte complex has different effects on the release rate of a water-soluble drug and a water-insoluable drug. Comparing the release rates shown in FIGS. 2 and 3, the presence of the bioactive polyelectrolyte complex in the polymer matrix decreases the release rate of the water-soluble drug and increases the release rate of the water-insoluble drug.

### EXAMPLE 4

Samples in this example were prepared by the same methods disclosed in Example 1, except chondroitin sulfate was replaced with heparin. To demonstrate that the bioactive polyelectrolyte complex was also controllably released from a drug delivery system containing drug, polymer, and bioactive polyelectrolyte complex, each coated stainless steel wire was incubated in saline solution containing 1% Tween-80 at 37°C. An aliquot of the solution was withdrawn at different time points and was analyzed for the amount of bioactive polyelectrolyte complex released into the solution. The amount of bivactive polyelectrolyte complex released was determined by measuring the biological activity of heparin using chromogenic substrate S2237.

The release rate of bioactive polyelectrolyte complex is shown in FIG. 4. Furthermore, FIG. 4 illustrates that the presence of a bioactive polyelectrolyte complex not only modulates the drug release rate of drug (as shown in Examples 1-3), but the bioactive polyelectrolyte complex itself is also controllably released over a period of time to exert an therapeutic function independent of the released drug. As shown in FIG. 4, approximately 40% of the available bioactive polyelectrolyte complex was released after 200 days.

### EXAMPLE 5

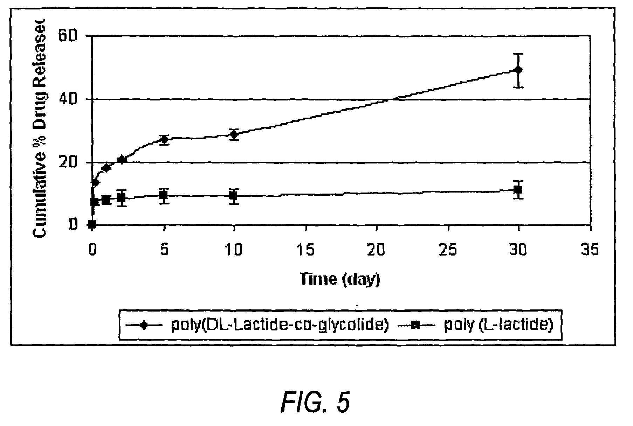

To demonstrate the compatibility of biodegradable polymers with the drug delivery compositions and the effect of different polymers on drug release rate, poly (DL-lactide-co-glycolide) or poly (L-lactide) was used as the polymer in the composition. Sample preparation techniques and methods of determining the drug release rates were the same as described in Examples 1 and 2, except poly (DL-lactide-co-glycolide) or poly (L-lactide) were used as the polymer. As shown in FIG. 5, a drug delivery composition including poly (DL-lactide-co-glycolide) had an increased drug release rate as compared to poly (L-lactide).

### EXAMPLE 6

To illustrate the effect of the bioactive polyelectrolyte complex on physical properties of the polymeric matrix, the glass transition temperature of polybutylmethacrylate was compared to the glass transition temperature of polybutylmethacrylate and a complex formed from heparinoid and alkyldimethylbenzylammonium chloride at a mix ratio of 2:1. The samples were held at -30° C for 5 minutes and then heated to 75° C at 20° C/min. The samples were held at 75° C for 5 minutes and quench cooled to -30°C. After the samples were held at -30°C for 5 minutes, they were then heated to 70° C at 20° C/min under a nitrogen atmosphere. The samples were subjected to DSC thermographic analysis as per ISO 113572-2.

A significant reduction in glass transition temperature was shown for the sample containing the bioactive polyelectrolyte complex as compared to the sample not including the complex. More specifically, the glass transition temperature (which is an indicator of polymer elasticity) was approximately 21°C for a sample containing only polybutylmethacrylate. The glass transition temperature was approximately 11 °C for a sample containing the mixture of polybutylmethacrylate and bioactive polyelectrolyte complex. The drop in the glass transition temperature shows that the rigidity of the polymer decreases due to the presence of the bioactive polyelectrolyte complex. Also, a single glass transition temperature also suggests that the bioactive polyelectrolyte complex is compatible with polybutylmethacrylate (whereas incompatible components typically results in two glass transition temperatures).

The various embodiments described above are provided by way of illustration only and should not be construed to limit the claimed invention. Those skilled in the art will readily recognize various modifications and changes that may be made to the claimed invention without following the example embodiments and applications illustrated and described herein.

## Claims

1. A drug delivery composition comprising:
a polymer;
one or more drugs dispersed within the polymer; and
a bioactive polyelectrolyte complex dispersed within the polymer that regulates the release of the drugs from the polymer, wherein the bioactive polyelectrolyte complex comprises a polyelectrolyte and an oppositely charged component, wherein the polyelectrolyte and/or the oppositely charged component is bioactive, or wherein the bioactive polyelectrolyte complex is formed from positively-charged and negatively-charged polyelectrolytes.

2. The drug delivery composition of claim 1, wherein the polyelectrolyte is selected from sodium salt of double and single stranded DNA; glycosaminoglycans, particularly dextran sulfate, chondroitin sulfate, dermatan sulfate, dermatan disulfate, heparan sulfate, keratan sulfate, hyaluronic acid, heparinoid, or heparin, anionic polysaccharides, xanthan gum, carrageenan, polyacrylic acid, poly(styrene sulfonate), polyamino acids, particularly poly(L-lysine), poly (L-arginine), poly (glutamic acid), poly(L-histidine), oligopeptides, insulin, mono-clonal antibodies, protamine sulfate, polyhexamethylene biguanide, chitosan, polyethyteneimine, polydimethyldiallylammonium chloride; poly(N-methyl-4-vinyl-pyridinium), poly (ionene-6,3) or quaternized polymers or copolymer of dimethylaminoethyl methacrylate.

3. The drug delivery composition of claim 1, wherein the oppositely charged component is selected from retinoic acid, compounds containing amino or amine functional groups, or cationic, anionic, or amphoteric surfactants.

4. The drug delivery composition of claim 3, wherein the compounds containing amino or amine functional groups are selected from amitriptyline, penicillian, amoxiciltian, tyramine, hexadimethrine bromide, tridodecyl amine, trimethyl-tetradecylammonium chloride, alkyldimethylbenzyl ammonium chloride, or tridodecylmethyl ammonium chloride.

5. The drug delivery composition of claim 1, wherein the bioactive polyelectrolyte complex comprises a glycosaminoglycan combined with a cationic salt.

6. The drug delivery composition of claim 1, wherein the bioactive polyelectrolyte complex is a complex of Vitamin A acid (retinoic acid) and poly(L-lysine); insulin and poly(methacrylic acid); insulin and hyaluronic acid; or heparin and di-methylalkylbenzyl ammonium chloride, preferably a complex of heparin and di-methylalkylbenzyl ammonium chloride

7. The drug delivery composition of claim 1, wherein the polymer comprises poly(lactic acid)s, poly(glycolic acid)s, poly(lactic acid-co-glycolic acid)s, polyanhydrides, polyorthoesters, polyetheresters, polycaprolactone, polyesteramides, polyolefins, polystyrene, polyvinyl acetate, acrylic polymers, polyethers, fluoropolymers, polyesters, polyamides, polyurethanes, silicone polymers, polydienes, cellulose and its derivatives, cross-linkable water-soluble polymers, blends or copolymers thereof.

8. The drug delivery composition of claim 1, wherein the polyelectrolyte complex concentration ranges from 0.1% (w/w) to 90% (w/w).

9. The drug delivery composition of claim 1, wherein the drug concentration ranges from 0.1% (w/w) to 70% (w/w).

10. The drug delivery composition of claim 1, wherein the drugs are selected from anti-inflammatory agents, antiproliferative agents, antineoplastic agents, anti-mitotic agents, anti-migratory agents, agents affecting extracellular matrix production and organization, agents promoting healing and re-endothelialization, anti-coagulants, anti-thrombotic agents, vascular cell growth promoters, vascular cell growth inhibitors, cholesterol-lowering agents, vasodilating agents, anesthetic agents, lubricants or humectants, anti-glaucoma agents, anti-cataract agents, anti-crustation agents, analgesics, antiasthmatics, antibiotics, antide-pressants, antidiabetics; antifungal agents, antihypertensive agents, anticancer agents, antianxiety agents, immunosuppressive agents, antimigraine agents, antianginal agents; antipsychotic agents; antimanic agents; antiarthritic agents, anti-gout agents; anticoagulants, thrombolytic agents, fibrinolytic agents; antiplatelet agents; anticonvulsants; antiparkinson agents; antihistamines; antiviral agents, antimicrobials, sedatives, hemorheologic agents, bronchodilators, steroidal compounds, hypoglycemic agents, hypolipidemic agents, proteins, nucleic acids, or vitamins.

11. The drug delivery composition of claim 1, wherein the drug is selected from the group consisisting of ibuprofen, salicylic acid, ticlopidine, clopidogrel, prasugrel, glycoprotein IIbIIIa inhibitor, statins, heparin, hirudin, urokinase, streptokinase, tissue plasminogen activator, nitric oxide, angiotensin converting enzyme inhibitor, dexamethsone, sirolimus, everolimus, zatarolimus, pimecrolimus, tacrolimus, biolimus, paclitaxel, estradiol, tranilast, tripidal, angiopeptin, brimonidine, timiolol, carbonic anhydrase inhibitors, their analogs and derivatives.

12. A medical device comprising: one or more surfaces, wherein at least one surface includes a biocompatible drug delivery composition as defined in anyone of claims 1 to 11.

13. The drug delivery composition of any one of claims 1 to 11 for use in administering the one or more drugs via subcutaneous, intramuscular, intraperitoneal, intradermal, intravenous, intra-arterial, intrathecal or intranasal applications, or via a medical device having a coating or a film that includes the drug delivery composition, or via a medical device having the drug delivery composition impregnated therein.

## Patentansprüche

1. Arzneimittelabgabezusammensetzung, umfassend:
ein Polymer;
ein oder mehrere Arzneimittel, dispergiert in dem Polymer, und
einen bioaktiven Polyelektrolytkomplex, dispergiert in dem Polymer, welcher die Freisetzung der Arzneimittel von dem Polymer reguliert, wobei der bioaktive Polyelektrolytkomplex ein Polyelektrolyt und eine entgegengesetzt geladene Komponente umfaßt, wobei der Polyelektrolyt und/oder die entgegengesetzt geladene Komponente bioaktiv ist, oder wobei der bioaktive Polyelektrolytkomplex aus positiv-geladenen und negativ-geladenen Polyelektrolyten gebildet ist.

2. Arzneimittelabgabezusammensetzung gemäß Anspruch 1, wobei der Polyelektrolyt aus einem Natriumsalz von doppel- und einzelsträngiger DNA, Glycosaminglycanen, insbesondere Dextransulfat, Chondroitinsulfat, Dermatansulfat, Dermatandisulfat, Heparansulfat, Keratansulfat, Hyaluronsäure, Heparinoid oder Heparin, anionischen Polysacchariden, Xanthangummi, Carrageenan, Polyacrylsäure, Poly(styrolsulfonat), Polyaminosäuren, insbesondere Poly(L-lysin), Poly(L-arginin), Poly(glutaminsäure), Poly(L-Histidin), Oligopeptiden, Insulin, monoklonalen Antikörpern, Protaminsulfat, Polyhexamethylenbiguanid, Chitosan, Polyethylenimin, Polydimethyldiallylammoniumchlorid, Poly(N-methyl-4-vinyl-pyridinium), Poly(ionen-6,3) oder quaternisierten Polymeren oder Copolymer von Dimethylaminomethacrylat, ausgewählt ist.

3. Arzneimittelabgabezusammensetzung gemäß Anspruch 1, wobei die entgegengesetzt geladene Komponente aus Retinolsäure, Verbindungen, enthaltend Amino- oder Amin-funktionale Gruppen oder kationischen, anionischen oder amphoteren grenzflächenaktiven Mitteln ausgewählt ist.

4. Arzneimittelabgabezusammensetzung gemäß Anspruch 3, wobei die Verbindungen, enthaltend amino- oder aminfunktionale Gruppen, aus Amitriptylin, Penicillin, Amoxicillin, Tyramin, Hexadimethrinbromid, Tridodecylamin, Trimethyltetradecylammoniumchlorid, Alkyldimethylbenzylammoniumchlorid oder Tridodecylmethylammoniumchlorid ausgewählt sind.

5. Arzneimittelabgabezusammensetzung gemäß Anspruch 1, wobei der bioaktive Polyelektrolytkomplex ein Glycosaminoglycan kombiniert mit einem kationischen Salz umfaßt.

6. Arzneimittelabgabezusammensetzung gemäß Anspruch 1, wobei der bioaktive Polyelektrolytkomplex ein Komplex von Vitamin A Säure (Retinolsäure) und Poly(L-lysin), Insulin und Poly(methacrylsäure), Insulin und Hyaluronsäure, oder Heparin und Dimethylalkylbenzylammoniumchlorid, vorzugsweise ein Komplex von Heparin und Dimethylalkylbenzylammoniumchlorid, ist.

7. Arzneimittelabgabezusammensetzung gemäß Anspruch 1, wobei das Polymer Poly(milchsäure)n), Poly(glykolsäure)n), Poly(milchsäure-co-glykolsäure)n), Polyanhydride, Polyorthoester, Polyetherester, Polycaprolacton, Polyesteramide, Polyolefine, Polystyrole, Polyvinylacetat, Acrylpolymere, Polyether, Fluorpolymere, Polyester, Polyamide, Polyurethane, Silikonpolymere, Polydiene, Cellulose und deren Derivate, vernetzbare wasserlösliche Polymere, Blends oder Copolymere davon, umfaßt.

8. Arzneimittelabgabezusammensetzung gemäß Anspruch 1, wobei die Polyelektrolytkomplexkonzentration im Bereich von 0,1% (Gew./Gew.) bis 90% (Gew./Gew.) ist.

9. Arzneimittelabgabezusammensetzung gemäß Anspruch 1, wobei die Arzneimittelkonzentration im Bereich von 0,1% (Gew./Gew.) bis 70% (Gew./Gew.) ist.

10. Arzneimittelabgabezusammensetzung gemäß Anspruch 1, wobei die Arzneimittel aus antiinflammatorischen Mitteln, antiproliferativen Mitteln, antineoplastischen Mitteln, antimitotischen Mitteln, antimigratorischen Mitteln, Mitteln, welche extrazelluläre Matrix-Produktion und Organisation berühren, Mittel, welche die Wundheilung und Re-Endothelialisation fördern, Antikoagulanzien, antithrombotischen Mitteln, vaskulären Zellwachstumspromotoren, vaskulären Zellwachstumsinhibitoren, Cholesterin-erniedrigenden Mitteln, vasodilatorischen Mitteln, anästhetischen Mitteln, Schmiermitteln oder Feuchthaltemitteln, Antiglaukomamitteln, Antikataraktmitteln, Antikrustationsmittel, Analgetika, Antiasthmatika, Antibiotika, Antidepressiva, Antidiabetika, antifugalen Mitteln, antihypertensiven Mitteln, Antikrebsmitteln, Anxiolytika, immunosuppressiven Mitteln, Antimigränemitteln, Antianginamitteln, antipsychotischen Mitteln, antimanischen Mitteln, antiarthritischen Mitteln, Antigichtmitteln, Antikoagulanzien, thromobolytischen Mitteln, fibrinolytischen Mitteln, Antiplättchenmitteln, Antikonvulsiva, Antiparkinsonmitteln, Antihistaminen, antiviralen Mitteln, antimikrobischen Mitteln, Seditativen, hämorheologischen Mitteln, Bronchodilatoren, steroidalen Verbindungen, hypoglykämischen Mitteln, hypolipidemischen Mitteln, Proteinen, Nukleinsäuren oder Vitaminen ausgewählt sind.

11. Arzneimittelabgabezusammensetzung gemäß Anspruch 1, wobei das Arzneimittel aus der Gruppe, bestehend aus Ibuprofen, Salicylsäure, Ticlopidin, Clopidogrel, Prasugrel, Glycoprotein IIbIIIa Inhibitor, Statinen, Heparin, Hirudin, Urokinase, Streptokinase, Gewebe-Plasminogen-Aktivator, Stickoxid, Angiotensin umwandelndem Enzyminhibitor, Dexamethson, Sirolimus, Everolimus, Zatarolimus, Pimecrolimus, Tacrolimus, Biolimus, Paclitaxel, Östradiol, Tranilast, Tripidal, Angiopeptin, Brimonidin, Timolol, Carboanhydrase-Inhibitoren, deren Analoge und Derivate, ausgewählt ist.

12. Medizinische Vorrichtung, umfassend: ein oder mehrere Oberflächen, wobei mindestens eine Oberfläche eine biokompatible Arzneimittelabgabezusammensetzung, wie in einem der Ansprüche 1 bis 11 definiert, einschließt.

13. Arzneimittelabgabezusammensetzung gemäß einem der Ansprüche 1 bis 11 zur Verwendung in der Verabreichung der einen oder mehreren Arzneimittel über subkutane, intramuskuläre, intraperitonale, intradermale, intravenöse, intraarterielle, intrathekale oder intranasale Anwendungen, oder über eine medizinische Vorrichtung mit einer Beschichtung oder einem Film, welcher die Arzneimittelabgabezusammensetzung einschließt, oder über eine medizinische Vorrichtung, welche die Arzneimittelabgabevorrichtung darin imprägniert aufweist.

## Revendications

1. Composition d'administration de médicaments comprenant :
un polymère ;
un ou plusieurs médicaments dispersés à l'intérieur du polymère ; et
un complexe de polyélectrolyte bioactif dispersé à l'intérieur du polymère qui régule la libération des médicaments hors du polymère, dans laquelle le complexe de polyélectrolyte bioactif comprend un polyélectrolyte et un composant de charges opposées, dans laquelle le polyélectrolyte et/ou le composant de charges opposées sont bioactifs, ou dans laquelle le complexe de polyélectrolyte bioactif est formé de polyélectrolytes de charges positives et de charges négatives.

2. Composition d'administration de médicaments selon la revendication 1, dans laquelle le polyélectrolyte est choisi parmi un sel de sodium d'ADN bi- et mono-caténaire ; le glycosaminoglycans, en particulier le sulfate de dextran, le sulfate de chondroïtine, le sulfate de dermatan, le disulfate de dermatan, le sulfate d'héparan, le sulfate de kératan, l'acide hyaluronique, l'héparinoïde ou l'héparine, les polysaccharides anioniques, la gomme xanthane, la carragénine, le poly(acide acrylique), le poly(sulfonate de styrène), les poly(acides aminés), en particulier la poly(L-lysine), la poly(L-arginine), le poly(acide glutamique), la poly(L-histidine), les oligopeptides, l'insuline, les anticorps monoclonaux, le sulfate de protamine, le polyhexaméthylène biguanide, le chitosan, la polyéthylèneimine, le poly(chlorure de diméthyldiallylammonium), le poly(N-méthyl-4-vinyl-pyridinium), la poly(ionène-6,3) ou les polymères ou les copolymères quaternisés de méthacrylate de diméthylaminoéthyle.

3. Composition d'administration de médicaments selon la revendication 1, dans laquelle le composant de charges opposées est choisi parmi l'acide rétinoïque, les composés contenant des groupes fonctionnels amino ou amine ou les tensioactifs cationiques, anioniques ou amphotères.

4. Composition d'administration de médicaments selon la revendication 3, dans laquelle les composés contenant des groupes fonctionnels amino ou amine sont choisis parmi l'amitriptyline, le pénicillian, l'amoxicillian, la tyramine, le bromure d'hexadiméthrine, la tridocécylamine, le chlorure de triméthyl-tétradécylammonium, le chlorure d'alkyldiméthylbenzyle ou le chlorure de tridodécylméthylammonium.

5. Composition d'administration de médicaments selon la revendication 1, dans laquelle le complexe de polyélectrolyte bioactif comprend un glycosaminoglycan combiné avec un sel cationique.

6. Composition d'administration de médicaments selon la revendication 1, dans laquelle le complexe de polyélectrolyte bioactif est un complexe d'acide de vitamine A (acide rétinoïque) et de poly(L-lysine) ; d'insuline et de poly(acide méthacrylique) ; d'insuline et d'acide hyaluronique ; ou d'héparine et de chlorure de diméthylalkylbenzylammonium, de préférence un complexe d'héparine et de chlorure de diméthylalkylbenzylammonium.

7. Composition d'administration de médicaments selon la revendication 1, dans laquelle le polymère comprend des poly(acides lactiques), des poly(acides glycoliques), des copolymères d'acide lactique-acide glycolique, des polyanhydrides, des polyorthoesters, des polyétheresters, de la polycaprolactone, des polyestéramides, des polyoléfines, du polystyrène, du poly(acétate de vinyle), des polymères acryliques, des polyéthers, des polymères fluorés, des polyesters, des polyamides, des polyuréthanes, des polymères de silicone, des polydiènes, de la cellulose et ses dérivés, des polymères solubles dans l'eau réticulables, des mélanges ou des copolymères de ceux-ci.

8. Composition d'administration de médicaments selon la revendication 1, dans laquelle la concentration du complexe de polyélectrolyte s'échelonne de 0,1 % (en poids) à 90 % (en poids).

9. Composition d'administration de médicaments selon la revendication 1, dans laquelle la concentration des médicaments s'échelonne de 0,1 % (en poids) à 70 % (en poids).

10. Composition d'administration de médicaments selon la revendication 1, dans laquelle les médicaments sont choisis parmi les agents anti-inflammatoires, les agents antiprolifératifs, les agents antinéoplasiques, les agents antimitotiques, les agents anti-migration, les agents affectant la production et l'organisation de la matrice extracellulaire, les agents favorisant la cicatrisation et une nouvelle endothélialisation, les anticoagulants, les agents anti-thrombotiques, les promoteurs de croissance cellulaire vasculaire, les inhibiteurs de croissance cellulaire vasculaire, les agents abaissant le taux de cholestérol, les agents vasodilatateurs, les agents anesthésiques, les lubrifiants ou les humidifiants, les agents anti-glaucome, les agents anti-cataracte, les agents anti-croute, les analgésiques, les antiasthmatiques, les antibiotiques, les antidépresseurs, les antidiabétiques, les agents antifongiques, les agents antihypertenseurs, les agents anticancéreux, les agents anti-anxiété, les agents immunosuppresseurs, les agents antimigraineux, les agents anti-angine, les agents antipsychotiques, les agents antimaniques, les agents antiarthritiques, les agents antigoutteux, les anticoagulants, les agents thrombotiques, les agents fibrinolytiques, les agents antiplaquettaires, les agents anticonvulsivants ; les agents antiparkinsoniens ; les anti-histamines ; les agents antiviraux, les agents antimicrobiens, les sédatifs, les agents hémorhéologiques, les bronchodilatateurs, les composés stéroïdiens, les agents hypoglycémiques, les agents hypolipidémiques, les protéines, les acides nucléiques ou les vitamines.

11. Composition d'administration de médicaments selon la revendication 1, dans laquelle le médicament est choisi dans le groupe constitué de l'ibuprofène, de l'acide salicylique, de la ticlopidine, du clopidogrel, du prasugrel, d'un inhibiteur de glycoprotéine IIbIIIa, des statines, de l'héparine, de l'hirudine, de l'urokinase, de la streptokinase, de l'activateur tissulaire du plasminogène, de l'oxyde nitrique, d'un inhibiteur d'enzyme de conversion de l'angiotensine, de la dexamethsone, du sirolimus, de l'évérolimus, du zatarolimus, du pimécrolimus, du tacrolimus, du biolimus, du paclitaxel, de l'estradiol, du tranilast, du tripidal, de l'angiopeptine, de la brimonidine, du timolol, des inhibiteurs de l'anhydrase carbonique, leurs analogues et leurs dérivés.

12. Dispositif médical comprenant : une ou plusieurs surfaces, dans lequel au moins une surface comprend une composition biocompatible d'administration de médicaments telle que définie dans l'une quelconque des revendications 1 à 11.

13. Composition d'administration de médicaments selon l'une quelconque des revendications 1 à 11 destinée à une utilisation pour l'administration du ou de plusieurs médicaments par application sous-cutanée, intramusculaire, intrapéritonéale, intradermique, intraveineuse, intra-artérielle, intrathécale ou intranasale, ou par l'intermédiaire d'un dispositif médical portant un revêtement ou un film qui comprend la composition d'administration de médicaments ou par l'intermédiaire d'un dispositif médical dans lequel est imprégnée la composition d'administration de médicaments.
